# EUROPEAN PATENT APPLICATION

(11) **EP 4 129 209 A1**
(43) Date of publication of application: **08.02.2023**
(21) Application number: 21780910.2
(22) Date of filing: 10.03.2021
(51) Int. Cl.: A61B 17/12

(54) **MEDICAL INSTRUMENT SET, DELIVERY SYSTEM, AND EMBOLUS DELIVERY MEDICAL SYSTEM**

(30) Priority: 31.03.2020 JP 2020062225
(71) Applicant: TERUMO Kabushiki Kaisha, Tokyo 151-0072 (JP)
(72) Inventor: SHIBATA, Hideaki, Ashigarakami-gun, Kanagawa 259-0151 (JP); ITOH, Yuki, Ashigarakami-gun, Kanagawa 259-0151 (JP); IKUNO, Eri, Ashigarakami-gun, Kanagawa 259-0151 (JP)
(74) Representative: Casalonga
(86) International application number: PCT/JP2021/009410
(87) International publication number: WO 2021/199973

(57) **Abstract**

[Problem] To displace an amount of curvature of a curved section provided at the tip of an insertion part that delivers an embolus into an aneurysm, when inserting or removing thereof, to facilitate insertion/removal operations. [Solution] The present invention comprises an elongated and hollow delivery catheter 20 that: includes an elongated main body 21, and a curved section 26 which extends at the distal side of the main body 21 and curves so as to gradually move away from a center axis of the main body 21; has a lumen 20a that continues from the distal end of the curved section 26 toward the base end of the main body 21; and delivers an embolus into an aneurysm through the lumen 20a. The invention also comprises an elongated correcting member 30 that is inserted into the lumen 20a of the delivery catheter 20 and that displaces the curved section 26 so that an amount of curvature of the curved section 26 after insertion is less than that before insertion.

## Description

### Technical Field

The present invention relates to a medical instrument set, a delivery system including the medical instrument set, and an embolus delivery medical system including the delivery system.

### Background Art

There is no drug treatment to prevent an aneurysm (aortic aneurysm) generated in an aorta of a patient from increasing in diameter and rupturing, and a surgical treatment (surgery) is generally performed on an aneurysm having a diameter with a risk of rupture. Further, in a surgery of the aortic aneurysm of the related art, artificial blood vessel substitution surgery in which an artificial blood vessel (stent graft) is transplanted by laparotomy or thoracotomy has been a mainstream, and in recent years, application of lower invasive stent graft interpolation (for endovascular aneurysm repair: EVAR) has been rapidly expanding.

For example, in stent graft interpolation for an abdominal aortic aneurysm (AAA), a catheter having a stent graft attached to a distal end thereof is inserted from a peripheral blood vessel of the patient, and the stent graft is opened and indwelled in an aneurysm target lesion, thereby blocking a blood flow to the aneurysm and preventing the aneurysm from rupturing.

In general, a stent graft used in the stent graft interpolation has a structure in which two types of members, that is, a "main body portion" including bifurcated portions bifurcated into a substantially Y-shape and "limb portions" attached to the bifurcated portions and attached to a right iliac artery and a left iliac artery, respectively, are assembled.

Therefore, in the stent graft interpolation, so-called "endoleak" in which the blood flow remains in the aneurysm may occur due to blood leakage from a periphery of the stent graft due to insufficient adhesion of the interpolated stent graft, backflow of blood from a thin blood vessel (side branch blood vessel) branched from the aneurysm, or the like. In this case, since a pressure is applied to a wall of the aneurysm by the blood flow entering the aneurysm, there is a potential risk of rupture of the aneurysm.

The following PTL 1 discloses a device including a catheter capable of holding a compressed relatively elongated sponge (embolus) in a lumen of the catheter and a plunger for pushing the embolus held in the catheter into an aneurysm filled with blood in order to block blood flow remaining in an aortic aneurysm due to endoleak. Since the sponge used in this device expands immediately when exposed to the blood, the sponge is pushed into the aneurysm and expands when absorbing the blood in the aneurysm, and the sponge is indwelled in the aneurysm in this state to block the blood flow and prevent rupture.

### Citation List

### Patent Literature

PTL 1: US Patent No. 9561096

### Summary of Invention

### Technical Problem

In endoleak embolization performed in PTL 1, when the embolus to be used enters slight gaps that can be generated at connection portions between bifurcated portions and limb portions after a stent graft is indwelled, the connection portions may be expanded when the embolus is expanded to cause the endoleak.

Further, in order to block the blood flow from a side branch blood vessel branched from the aneurysm, the embolus is indwelled in the vicinity of the side branch blood vessel, and when the embolus enters from a communication port between a wall portion of the aneurysm and the side branch blood vessel, there is a concern that distal occlusion may occur due to the embolus flowing through the blood vessel.

Since the device disclosed in PTL 1 includes a curved portion at a distal end of an insertion portion (catheter) that delivers the embolus into the aneurysm, a push-out direction of the embolus in the aneurysm is controlled to some extent by the surgeon changing a direction of the distal end of the curved portion while rotating the insertion portion.

However, in the device of PTL 1, since a curved state of the curved portion before and after the insertion portion is inserted into a living body lumen does not change, for example, when the insertion portion is removed, there is a problem that the curved portion is caught in the living body lumen and is difficult to be removed.

At least one embodiment of the invention has been made in view of the above circumstances, and specifically, an object thereof is to provide a medical instrument set, a delivery system, and an embolus delivery medical system capable of facilitating an insertion and removal operation by displacing, at a time of insertion or removal, an amount of curvature of a curved portion provided at a distal end of an insertion portion that delivers an embolus into an aneurysm.

### Solution to Problem

A medical instrument set according to the present embodiment includes: an elongated hollow delivery catheter including an elongated main body, a curved portion that is provided to extend to a distal end side of the main body and curved so as to gradually move away from a central axis of the main body, and a lumen that communicates from a distal end side of the curved portion toward a proximal end side of the main body, and configured to deliver an embolus into an aneurysm via the lumen; and an elongated correcting member configured to displace the curved portion such that an amount of curvature of the curved portion after the insertion becomes smaller than that before the insertion by being inserted into the lumen of the delivery catheter.

A delivery system according to the present embodiment includes: the medical instrument set; and an elongated delivery pusher configured to push the embolus loaded in the lumen of the delivery catheter out of the lumen into the aneurysm.

An embolus delivery medical system according to the present embodiment includes: an embolus filled catheter including a lumen for filling and insertable into the lumen of the delivery catheter in a state in which the lumen for filling is filled with the embolus; and an elongated extrusion pusher configured to push the embolus out of the lumen for filling.

### Advantageous Effect of Invention

According to at least one embodiment of the invention, the amount of curvature of the curved portion provided at the distal end of the insertion portion that delivers the embolus into the aneurysm can be displaced at a time of insertion or removal to facilitate an insertion and removal operation.

### Brief Description of Drawings

FIG. 1 is a diagram showing a configuration of a medical instrument set and a configuration of a delivery set according to the present embodiment.
FIG. 2 is a diagram showing a configuration of an embolus delivery medical system according to the present embodiment.
FIG. 3 is a diagram showing an assembled state of the medical instrument set according to the present embodiment.
FIG. 4 is a cross-sectional view showing a state in which an embolus is conveyed in a delivery system according to the present embodiment.
FIG. 5 is a diagram showing a state in which a curved portion of an insertion portion is corrected by a correcting member in the medical instrument set according to the present embodiment.
FIG. 6A is a diagram showing an operation example of the embolus delivery medical system according to the present embodiment and showing a state in which the insertion portion is delivered into an aneurysm.
FIG. 6B is a diagram showing an operation example of the embolus delivery medical system according to the present embodiment and showing a state in which a stent graft is deployed in the aneurysm.
FIG. 6C is a diagram showing an operation example of the embolus delivery medical system according to the present embodiment and showing a state in which the embolus is pushed out of the insertion portion.
FIG. 6D is a diagram showing an operation example of the embolus delivery medical system according to the present embodiment and showing a state in which the embolus is indwelled in the aneurysm.
FIG. 6E is a diagram showing an operation example of the embolus delivery medical system according to the present embodiment and showing a state in which the embolus absorbs a liquid in the aneurysm and swells.
FIG. 7 is a diagram showing another aspect example (first modification) of the correcting member of the medical instrument set according to the present embodiment.
FIG. 8A is a cross-sectional view showing a state in which an embolus is conveyed by the correcting member according to the first modification.
FIG. 8B is a cross-sectional view showing a state in which a curved portion is corrected by the correcting member according to the first modification.
FIG. 9 is a diagram showing another aspect example (second modification) of the correcting member of the medical instrument set according to the present embodiment. Description of Embodiments

Hereinafter, an embodiment of the invention will be described in detail with reference to the drawings. The embodiment shown here is an example for embodying a technical idea of the invention, and does not limit the invention. Further, other aspects, examples, operational techniques, and the like that can be implemented by those skilled in the art without departing from the gist of the invention are all included in the scope and the gist of the invention, and are included in the invention described in the claims and the scope of equivalents thereof.

Further, in the drawings attached to the present specification, for convenience of illustration and understanding, a scale, an aspect ratio, a shape, and the like may be changed from actual ones and may be schematically expressed as appropriate, and the drawings are just examples and do not limit the interpretation of the invention.

An object of a medical instrument set 100, a delivery system 200, and an embolus delivery medical system 300 according to the present embodiment is to provide a curved portion 26 that is curved at a predetermined curvature with respect to a central axis of a main body 21 on a distal end side of the main body 21 of a delivery catheter 20 that delivers an embolus 10 into a tumor in a blood vessel (for example, an aneurysm) to control a push-out direction of the embolus 10 while operating the delivery catheter 20, allow the embolus 10 to be indwelled at an appropriate position in the aneurysm, and displace the curved portion 26 such that a curved state of the curved portion 26 becomes close to a substantially straight state at a time of insertion or removal of a delivery catheter 20, thereby smoothly performing an insertion and removal operation.

The medical instrument set 100, the delivery system 200, and the embolus delivery medical system 300 according to the present embodiment may be applied to endoleak embolization for stent graft interpolation of an abdominal aortic aneurysm (AAA), which is an example of a treatment method for preventing the aneurysm generated in the blood vessel from rupturing. In addition, the treatment method in which the medical instrument set 100, the delivery system 200, and the embolus delivery medical system 300 according to the present embodiment are used is not limited to the endoleak embolization for the stent graft interpolation of the abdominal aortic aneurysm, and is also applicable to other intervention treatment methods for preventing the aneurysm generated in the blood vessel from rupturing.

In the present specification, in an operation direction of each part constituting the embolus delivery medical system 300, for example, a side on which the embolus 10 is to be conveyed into the aneurysm in a direction along an axial direction of the delivery catheter 20 is referred to as a "distal end side", and a side that is located on a side opposite to the distal end side and on which a surgeon performs an operation with hands (a side from which the delivery catheter 20 is removed) is referred to as a "proximal end side". In addition, a direction parallel to the axial direction of the delivery catheter 20 and in which a delivery pusher 40 advances (is inserted) or retreats (is removed) is referred to as an "advancing and retreating direction", and a direction along a circumferential direction (a direction around the axial direction of the delivery catheter 20) of the delivery catheter 20 is referred to as a "rotation direction".

### [Configuration]

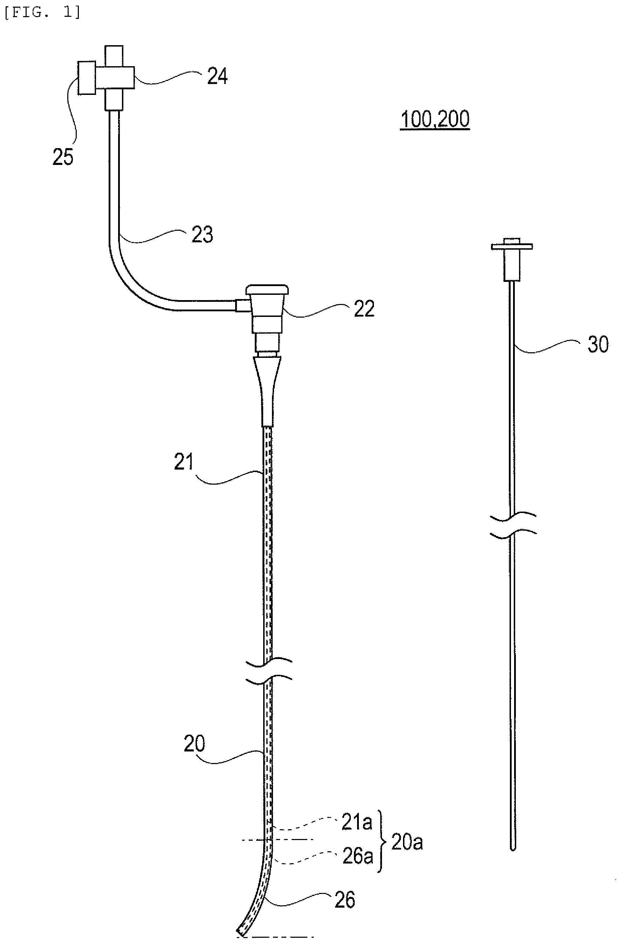

Configurations of the medical instrument set 100, the delivery system 200, and the embolus delivery medical system 300 according to the present embodiment will be described with reference to FIGS. 1 to 5 as appropriate.

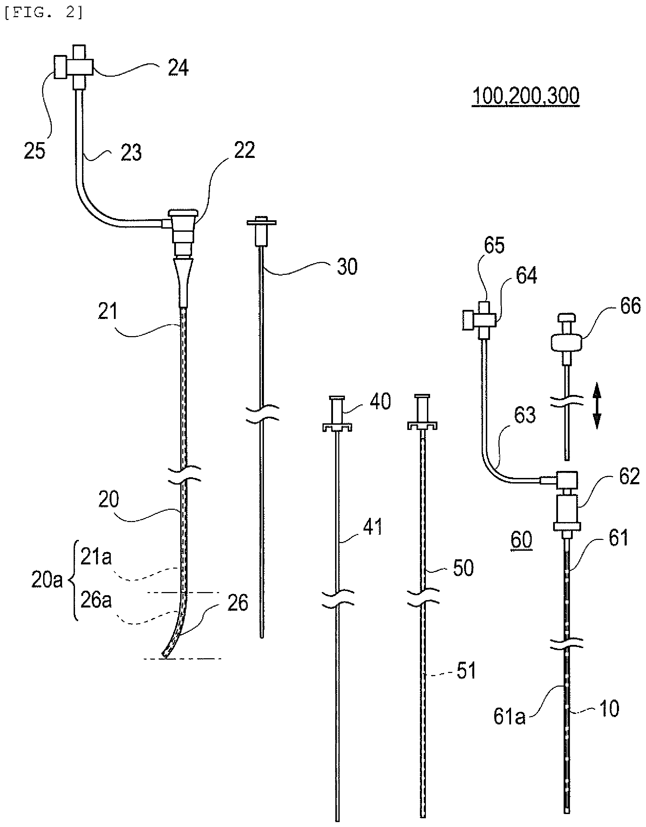

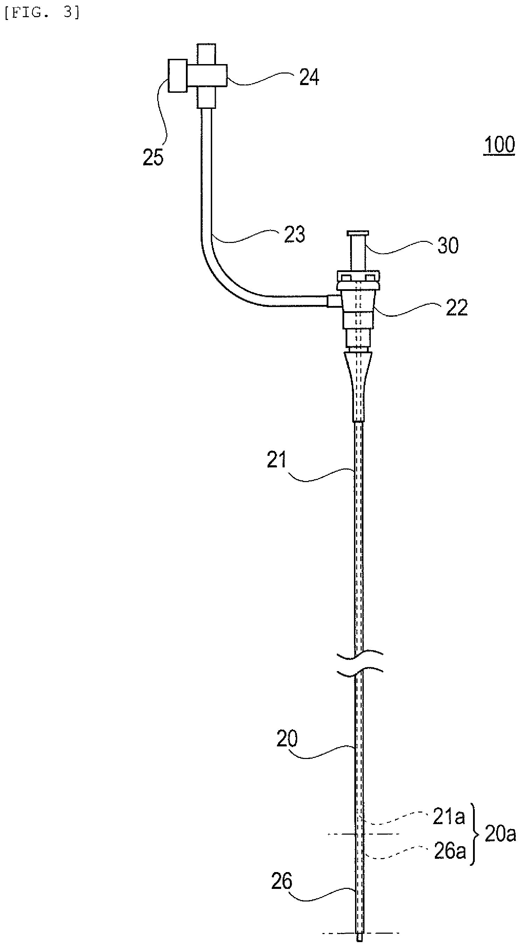

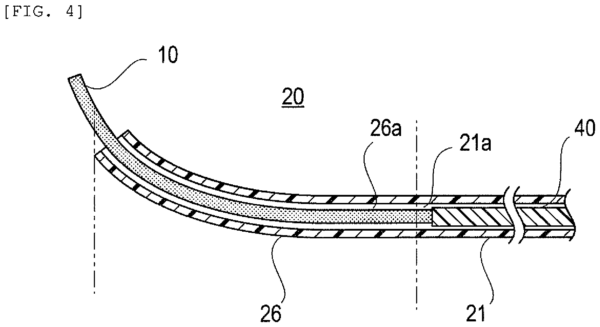

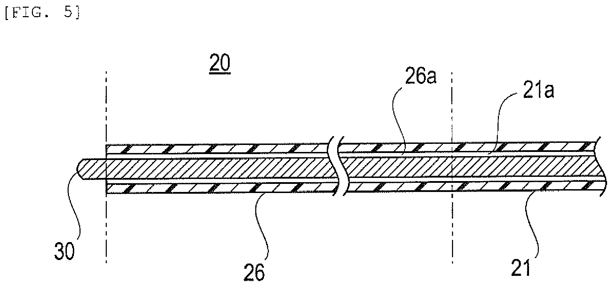

FIG. 1 shows parts constituting the medical instrument set 100 and the delivery system 200 according to the present embodiment, FIG. 2 shows parts constituting the embolus delivery medical system 300 according to the present embodiment, FIG. 3 shows an assembled state of the medical instrument set 100 according to the present embodiment, FIG. 4 shows a state in which the embolus 10 is conveyed in the delivery system 200, and FIG. 5 shows a state in which the curved portion 26 of the delivery catheter 20 is corrected by a correcting member 30.

First, the embolus 10 to be indwelled in the aneurysm using the medical instrument set 100, the delivery system 200, and the embolus delivery medical system 300 according to the present embodiment will be described.

### <Embolus>

The embolus 10 is indwelled in the aneurysm and expands by absorbing a liquid containing blood flowing into the aneurysm. After being loaded into the delivery catheter 20, the embolus 10 is conveyed into the aneurysm by the delivery pusher 40 to be described later.

The embolus 10 is an elongated fibrous linear body (umbilical member) made of an expandable material (polymer material (water-absorbing gel material) or the like) that expands by contacting with an aqueous liquid containing the blood under a physiological condition. In the present embodiment, the embolus 10 is an elongated umbilical member having a substantially circular cross-sectional shape in a direction orthogonal to a longitudinal direction. The cross-sectional shape of the embolus 10 is not particularly limited, and may be a polygonal shape in addition to the substantially circular shape as in the present embodiment.

Here, the "physiological condition" means a condition having at least one environmental characteristic in a body or a body surface of a mammal (for example, human). Such a characteristic include an isotonic environment, a pH buffering environment, an aqueous environment, a pH near neutral (about 7), or a combination thereof. The "aqueous liquid" includes, for example, body fluids of a mammal (for example, humans) such as isotonic fluid, water, blood, spinal fluid, plasma, serum, glass body fluid, and urine. A size (total length and width dimension) of the embolus 10 is not particularly limited, and can be appropriately set according to a size of the aneurysm to which the embolus 10 is to be indwelled, a thickness of a side branch blood vessel such as inferior mesenteric arteryor or a lumbar artery interlocked to the aneurysm, and the like.

A constituent material of the embolus 10 is not particularly limited as long as it is a material that expands by absorbing at least a liquid such as blood, and has no (or extremely low) harmful effect on a human body even in a state of being indwelled in the aneurysm. In addition, the embolus 10 may be added with a visualization material whose existing position in a living body can be confirmed by a confirmation method such as an X-ray, a fluorescent X-ray, an ultrasound, a fluorescence method, an infrared ray, or an ultraviolet ray.

### <Medical Instrument Set>

The configuration of the medical instrument set 100 according to the present embodiment will be described. As shown in FIG. 1, the medical instrument set 100 according to the present embodiment includes the delivery catheter 20 and the correcting member 30.

### (Delivery Catheter)

The delivery catheter 20 is, for example, a catheter formed with a hole (lumen 20a) inserted from the distal end side to the proximal end side along the axial direction is formed, and functions as a tubular introduction path for delivering the embolus 10 into the aneurysm. The lumen 20a of the delivery catheter 20 includes a lumen 21a provided in the main body 21 and a lumen 26a provided in the curved portion 26, and the embolus 10 is loaded by an embolus filled catheter set 60 to be described later. The main body 21 of the delivery catheter 20 can be penetrated by a main body of an insertion assisting member 50 over a total length of the main body 21. Therefore, a length of the main body 21 in the axial direction is at least shorter than that of the main body of the insertion assisting member 50.

A constituent material of the main body 21 is not particularly limited as long as the constituent material is flexible and is relatively softer than that of the correcting member 30. As examples of the constituent material of the main body 21, a resin material such as a polymer material including polyolefin (for example, polyethylene, polypropylene, polybutene, an ethylene-propylene copolymer, an ethylene-vinyl acetate copolymer, an ionomer, or a mixture of two or more types thereof), a polyolefin elastomer, crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof can be suitably used.

The delivery catheter 20 includes a hub 22 interlocked to a proximal end side of the main body 21, and a flexible tube 23 having one end connected to a proximal end side of the hub 22 and the other end connected to a three-way stopcock 24.

The hub 22 is an intermediate member that includes a lumen that allows the main body 21 and the tube 23 to communicate with each other, and allows a fluid (such as physiological saline for priming) flowing from the three-way stopcock 24 to flow to the main body 21 via the tube 23. The correcting member 30 and the insertion assisting member 50 are inserted through the hub 22.

A constituent material of the hub 22 is not particularly limited as long as the constituent material is a hard material such as a hard resin. As examples of the constituent material of the hub 22, polyolefin such as polyethylene or polypropylene, polyamide, polycarbonate, polystyrene, or the like can be suitably used.

A hemostasis valve (not shown) is attached into the proximal end side of the hub 22. As the hemostasis valve, for example, a substantially elliptical film-like (disk-shaped) valve body made of silicone rubber, latex rubber, butyl rubber, isoprene rubber, or the like, which is an elastic member, may be used.

The one end of the tube 23 is interlocked to the proximal end side of the hub 22, and the other end is connected to a port 25 of the three-way stopcock 24. The tube 23 is a conduit through which a liquid such as physiological saline flowing out from a priming syringe (not shown) interlocked to the port 25 flows.

The tube 23 is not particularly limited as long as it is a resin material having a certain degree of flexibility. As examples of a constituent material of the tube 23, polyolefin such as polyethylene, polypropylene, and ethylene-propylene copolymer, polyester such as polyethylene terephthalate, polystyrene, polyvinyl chloride, and the like can be suitably used.

The three-way stopcock 24 communicates with the lumen of the hub 22 and the lumen 21a of the main body 21 via the tube 23. In addition to a proximal end side of the tube 23, a priming syringe for priming the lumen 21a of the main body 21 and a liquid injection syringe for injecting a contrast agent, a drug, or the like can be connected to the port 25 of the three-way stopcock 24.

The delivery catheter 20 further includes the curved portion 26 extending from a distal end side of the main body 21. The curved portion 26 is provided to extend from a distal end of the main body 21, and is gradually curved in a direction (a radial direction with respect to the central axis of the main body 21) away from the central axis of the main body 21 from the proximal end side toward the distal end side. The curved portion 26 has a hole (lumen 26a) that communicates from the distal end side toward the proximal end side. The lumen 26a communicates with the lumen 21a of the main body 21, and functions as the lumen 20a of the delivery catheter 20. In the present embodiment, in FIG. 1, a range divided by two-dot chain lines is the curved portion 26.

The curved portion 26 is maintained in the curved state at least when delivered into the aneurysm, and can be displaced to a substantially straight state by the correcting member 30 at a time of insertion and removal of the delivery catheter 20. When the curved portion 26 is delivered in the aneurysm through the living body lumen, the curved portion 26 does not interfere with delivery because the correcting member 30 is inserted and curving becomes gentle, and when the curved portion 26 reaches the aneurysm, the correcting member 30 is removed and the curved portion 26 returns to an original curved state.

A constituent material of the curved portion 26 is not particularly limited as long as the material is flexible and can be displaced to the substantially straight state by the correcting member 30 at the time of the insertion and removal of the delivery catheter 20 while the curved state is maintained when the curved portion 26 is delivered into the aneurysm. As examples of the constituent material of the curved portion 26, a resin material such as a polymer material including polyolefin (for example, polyethylene, polypropylene, polybutene, an ethylene-propylene copolymer, an ethylene-vinyl acetate copolymer, an ionomer, or a mixture of two or more types thereof), a polyolefin elastomer, crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof can be suitably used.

As shown in FIG. 4, when the embolus 10 loaded in the delivery catheter 20 is conveyed by the delivery pusher 40, the embolus 10 is pushed out of the distal end side along the lumen 26a of the curved portion 26. Then, an opening on the distal end side of the curved portion 26 serving as an ejection port of the embolus 10 can be directed in a predetermined radial direction with respect to a rotation center (central axis) of the delivery catheter 20 by rotating the delivery catheter 20 in the rotation direction. Accordingly, a direction of the opening of the curved portion 26 is changed, and therefore the embolus 10 is indwelled at an appropriate position by directing a push-out direction of the embolus 10 to an appropriate direction in the aneurysm.

### (Correcting Member)

The correcting member 30 is a rod-shaped member that is inserted into the delivery catheter 20 at the time of the insertion and removal of the delivery catheter 20, and displaces an amount of curvature of the curved portion 26. When being inserted into the delivery catheter 20, the correcting member 30 displaces the amount of curvature (degree of curving) of the curved portion 26 such that the curved portion 26 becomes closer to the substantially straight state than that before the insertion. That is, when comparing before and after the insertion of the correcting member 30 into the delivery catheter 20, the degree of curving of the curved portion 26 after the insertion of the correcting member 30 is gentler than the degree of curving before the insertion of the correcting member 30. In order to improve ease at the time of the insertion and removal of the delivery catheter 20, the correcting member 30 preferably corrects the curved portion 26 such that the curved state of the curved portion 26 becomes the substantially straight state after the insertion of the correcting member 30.

A constituent material of the correcting member 30 is not particularly limited as long as the material is flexible and is harder than the delivery catheter 20 such that the curved state of the curved portion 26 can be corrected to the substantially straight state when the correcting member 30 is inserted into the delivery catheter 20. As examples of the constituent material of the correcting member 30, a resin material including polyolefin such as polyethylene and polypropylene, polyester such as polyamide and polyethylene terephthalate, a fluorous resin such as ETFE, polyetheretherketone (PEEK), and polyimide, and a metal material such as a shape memory alloy, stainless steel, tantalum, titanium, platinum, gold, and tungsten can be suitably used.

As shown in FIG. 3, by inserting and assembling the correcting member 30 into the delivery catheter 20, the curved state (for example, the state shown in FIG. 4) of the curved portion 26 is corrected, and the curved portion 26 is displaced to the substantially straight state as shown in FIG. 5. When the delivery catheter 20 is inserted and removed in this state, the insertion and removal operation of the delivery catheter 20 can be smoothly performed without the curved portion 26 being caught in the living body lumen. When the correcting member 30 is removed again in the state shown in FIG. 5, a shape of the curved portion 26 returns from the substantially straight state to the original curved state.

The correcting member 30 may be used at least when the delivery catheter 20 is removed. Further, when the correcting member 30 is used when the delivery catheter 20 is inserted into the living body lumen, since the insertion assisting member 50 performs the same function as the correcting member 30, a configuration of the insertion assisting member 50 may be unnecessary.

As described above, the medical instrument set 100 according to the present embodiment includes: the delivery catheter 20 including the main body 21 and the curved portion 26 that is provided to extend to the distal end side of the main body 21 and curved so as to gradually move away from the central axis of the main body 21, and configured to deliver the embolus 10 into the aneurysm via the lumen 20a that communicates from the distal end side of the curved portion 26 toward the proximal end side of the main body 21; and the correcting member 30 configured to displace the curved portion 26 such that the amount of curvature of the curved portion 26 after the insertion becomes smaller than that before the insertion by being inserted into the delivery catheter 20.

Therefore, in the medical instrument set 100, when the delivery catheter 20 is inserted into or removed from the living body lumen, the correcting member 30 can be inserted into the delivery catheter 20 to correct the curved state of the curved portion 26 so as to be the substantially straight state, and thus the delivery catheter 20 can be smoothly inserted into or removed from the living body lumen while preventing the curved portion 26 from being caught in the living body lumen at the time of the insertion and removal. Further, since the delivery catheter 20 includes the curved portion 26 at the distal end, the push-out direction of the embolus 10 can be directed to the radial direction with respect to the rotation center (central axis) of the delivery catheter 20 by rotating the delivery catheter 20 in the rotation direction. Therefore, the embolus 10 can be indwelled at an appropriate position in accordance with a situation (a state of the aneurysm, an implantation position of a stent graft SG, a branched state of the side branch blood vessel, and the like) of a surgery.

### <Delivery System>

Next, the configuration of the delivery system 200 according to the present embodiment will be described. As shown in FIG. 2, the delivery system 200 according to the present embodiment includes the delivery pusher 40 in addition to the medical instrument set 100.

### (Delivery Pusher)

The delivery pusher 40 includes a rod-shaped main body 41, and when a predetermined operation is performed in a state in which a proximal end side is gripped by the surgeon, the delivery pusher 40 conveys the embolus 10 loaded in the delivery catheter 20 into the aneurysm. Specifically, the delivery pusher 40 pushes out the embolus 10 loaded in the delivery catheter 20 to an outside by being subjected to a push-out operation along the advancing and retreating direction.

A constituent material of the main body 41 is not particularly limited as long as it is a material having appropriate hardness and flexibility such that the embolus 10 can be conveyed. As examples of the constituent material of the main body 41, a resin material such as a polymer material including polyolefin (for example, polyethylene, polypropylene, polybutene, an ethylene-propylene copolymer, an ethylene-vinyl acetate copolymer, an ionomer, or a mixture of two or more types thereof), a polyolefin elastomer, crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof, and a metal material such as a shape memory alloy, stainless steel, tantalum, titanium, platinum, gold, or tungsten can be suitably used.

As described above, the delivery system 200 according to the present embodiment includes the delivery pusher 40 for pushing out and conveying the embolus 10 loaded in the delivery catheter 20.

In the delivery system 200, the embolus 10 loaded in the delivery catheter 20 is pushed out and conveyed by the delivery pusher 40, and since the curved portion 26 is provided at the distal end of the delivery catheter 20, the push-out direction of the embolus 10 can be directed to the predetermined radial direction with respect to the rotation center (central axis) of the delivery catheter 20 by rotating the delivery catheter 20 in the rotation direction. Therefore, the surgeon can indwell the embolus 10 at the appropriate position by operating the delivery pusher 40 in a state in which the delivery catheter 20 is rotated by a predetermined amount in the rotation direction in accordance with the situation (the state of the aneurysm, the implantation position of the stent graft SG, the branched state of the side branch blood vessel, and the like) of the surgery.

### <Embolus Delivery Medical System>

Next, the configuration of the embolus delivery medical system 300 according to the present embodiment will be described. As shown in FIG. 2, the embolus delivery medical system 300 according to the present embodiment includes, in addition to the delivery system 200, the insertion assisting member 50 that delivers the delivery catheter 20 into the living body lumen, and the embolus filled catheter set 60 that loads the embolus 10 into the delivery catheter 20. As will be described later, the insertion assisting member 50 may be omitted.

### (Insertion Assisting Member)

The insertion assisting member 50 is formed with a guide wire lumen 51 that is inserted from a distal end side to a proximal end side along the axial direction, and is an assisting tool that assists insertion when the delivery catheter 20 is to be delivered into the aneurysm along a guide wire GW inserted into the living body lumen in advance. The insertion assisting member 50 is inserted and assembled into the delivery catheter 20 in order to prevent the delivery catheter 20 from being broken when the delivery catheter 20 is inserted into the living body lumen. The guide wire lumen 51 has an inner diameter smaller than that of the lumen 20a of the delivery catheter 20. Therefore, when the delivery catheter 20 is to be delivered into the aneurysm, an axial deviation of the delivery catheter 20 with respect to the guide wire GW can be reduced, leading to easier delivery. The insertion assisting member 50 can also function as the correcting member 30 that corrects the curved portion 26 of the delivery catheter 20.

A constituent material of the insertion assisting member 50 is not particularly limited as long as it is harder and more flexible than the delivery catheter 20. As examples of the constituent material of the insertion assisting member 50, a resin material including polyolefin such as polyethylene and polypropylene, polyester such as polyamide and polyethylene terephthalate, a fluorous resin such as ETFE, polyetheretherketone (PEEK), polyimide, and a metal material such as a shape memory alloy, stainless steel, tantalum, titanium, platinum, gold, and tungsten can be suitably used.

In the embolus delivery medical system 300, the insertion assisting member 50 may be omitted. In this case, the lumen 20a of the delivery catheter 20 also serves as the guide wire lumen 51.

### (Embolus Filled Catheter Set)

The embolus filled catheter set 60 is for loading the embolus 10 into the lumen 20a of the delivery catheter 20. The embolus filled catheter set 60 includes an embolus filled catheter 61 that is pre-filled with the embolus 10, and an elongated extrusion pusher 66 that is inserted into the embolus filled catheter 61 in order to push the embolus 10 accommodated in the embolus filled catheter 61 into the lumen 20a of the delivery catheter 20.

The embolus filled catheter 61 includes a filling catheter main body 61a filled with the embolus 10, a hub 62 to which a proximal end side of the filling catheter main body 61a is connected, and a flexible tube 63 having one end connected to a proximal end side of the hub 62 and the other end connected to a port 65 of a three-way stopcock 64.

The filling catheter main body 61a is a tubular member formed with a hole (lumen for filling) inserted from the distal end side to the proximal end side along the axial direction, and can accommodate the embolus 10 inserted from the distal end side. A length of the filling catheter main body 61a in an extending direction is appropriately defined, and it is sufficient that the filling catheter main body 61a has a length at least capable of accommodating the embolus 10. The filling catheter main body 61a is provided mainly in a state in which the embolus 10 is loaded in advance, and the embolus 10 accommodated in the filling catheter main body 61a may be accommodated in the filling catheter main body 61a by the surgeon or the like by gripping the embolus 10.

The filling catheter main body 61a is inserted into the hub 22 of the delivery catheter 20 in a state in which the embolus 10 is accommodated, and in this state, the embolus 10 is pushed out by the extrusion pusher 66. Accordingly, the embolus 10 accommodated in the filling catheter main body 61a is loaded into the lumen 21a of the delivery catheter 20. In addition, the filling catheter main body 61a can be used by pushing out the embolus 10 by the extrusion pusher 66 in a state of being inserted into the delivery catheter 20.

A constituent material of the filling catheter main body 61a is not particularly limited as long as it is a material having appropriate hardness and flexibility. As examples of the constituent material of the filling catheter main body 61a, a resin material such as a polymer material including polyolefin (for example, polyethylene, polypropylene, polybutene, an ethylene-propylene copolymer, an ethylene-vinyl acetate copolymer, an ionomer, or a mixture of two or more types thereof), a polyolefin elastomer, crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof, and a metal material such as a shape memory alloy, stainless steel, tantalum, titanium, platinum, gold, or tungsten can be suitably used.

The hub 62 is an intermediate member that includes a lumen that allows the filling catheter main body 61a and the tube 63 to communicate with each other, and allows a fluid (such as physiological saline for priming) flowing from the three-way stopcock 64 to flow to the filling catheter main body 61a via the tube 63. The extrusion pusher 66 is inserted through the hub 62, and the embolus 10 accommodated in the filling catheter main body 61a is pushed out. A constituent material of the hub 62 can be the same as the material exemplified as the constituent material of the hub 22 described above.

The one end of the tube 63 is interlocked to the proximal end side of the hub 62, and the other end is connected to the port 65 of the three-way stopcock 64. The tube 63 is a conduit through which a liquid such as physiological saline flowing out from a priming syringe (not shown) interlocked to the port 65 flows. A constituent material of the tube 63 can be the same as the material exemplified as the constituent material of the tube 23 described above.

The three-way stopcock 64 communicates with the lumen of the hub 62 and the lumen of the filling catheter main body 61a via the tube 63. In addition to a proximal end side of the tube 63, a priming syringe for priming the lumen of the filling catheter main body 61a can be connected to the port 65 of the three-way stopcock 64.

The extrusion pusher 66 is a rod-shaped member for pushing out the embolus 10 accommodated in the filling catheter main body 61a and loading the embolus 10 into the lumen 20a of the delivery catheter 20 by inserting through the hub 62. The extrusion pusher 66 is inserted from the hub 62 when the embolus 10 is loaded into the delivery catheter 20, and pushes out the embolus 10 in the lumen of the filling catheter main body 61a. Further, the extrusion pusher 66 also functions as the delivery pusher 40. For example, when the filling catheter main body 61a loaded with the embolus 10 is inserted into the delivery catheter 20, the extrusion pusher 66 can push out the embolus 10 into the filling catheter body 61a into the aneurysm like the delivery pusher 40.

A constituent material of the extrusion pusher 66 is not particularly limited as long as it is a material having rigidity that allows the embolus 10 to be pushed put. As examples of the constituent material of the extrusion pusher 66, polyolefin such as polyethylene and polypropylene, polyester such as polyamide and polyethylene terephthalate, a fluorous resin such as ETFE, polyetheretherketone (PEEK), polyimide, a metal such as a shape memory alloy, stainless steel, tantalum, titanium, platinum, gold, and tungsten, and the like can be suitably used.

In this way, the embolus delivery medical system 300 according to the present embodiment includes, in addition to the delivery system 200, the insertion assisting member 50 that assists delivery of the delivery catheter 20 into the aneurysm along the guide wire GW inserted into the living body lumen in advance by being assembled to the delivery catheter 20, and the embolus filled catheter set 60 that loads the embolus 10 into the delivery catheter 20, and the embolus 10 is loaded into the lumen 20a of the delivery catheter 20 by operating the extrusion pusher 66 in a state in which the embolus 10 is accommodated in the filling catheter main body 61a of the embolus filled catheter set 60.

Accordingly, even when the embolus 10 is relatively elongated, the embolus 10 can be smoothly loaded into the delivery catheter 20. Further, when the delivery catheter 20 is inserted into the living body lumen, the delivery catheter 20 can be smoothly inserted by performing the insertion in a state in which the insertion assisting member 50 is assembled.

### [Operation]

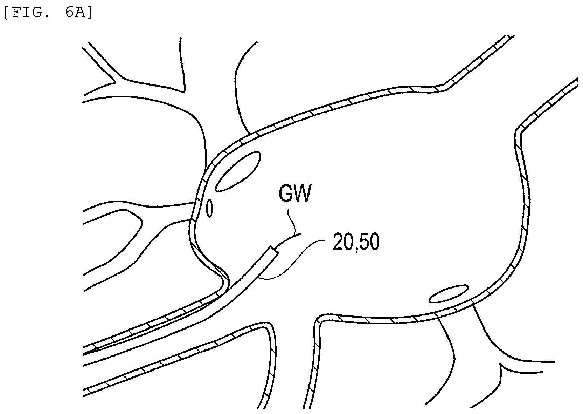

Next, an operation of the embolus delivery medical system 300 according to the present embodiment will be described. FIGS. 6A to 6E show main procedure steps of the endoleak embolization for the stent graft interpolation of the abdominal aortic aneurysm.

### (Step 1: Preparation Step)

First, in a state in which the insertion assisting member 50 is inserted into the delivery catheter 20, the surgeon percutaneously inserts the main body 21 into the living body lumen, and then removes the insertion assisting member 50. FIG. 6A shows a state in which the distal end of the delivery catheter 20 is delivered into the aneurysm along the guide wire GW. As shown in FIG. 6A, when the delivery catheter 20 is inserted into the aneurysm, the delivery catheter 20 is inserted in a state in which the insertion assisting member 50 is assembled.

### (Step 2: Stent Graft Deploying and Indwelling Step)

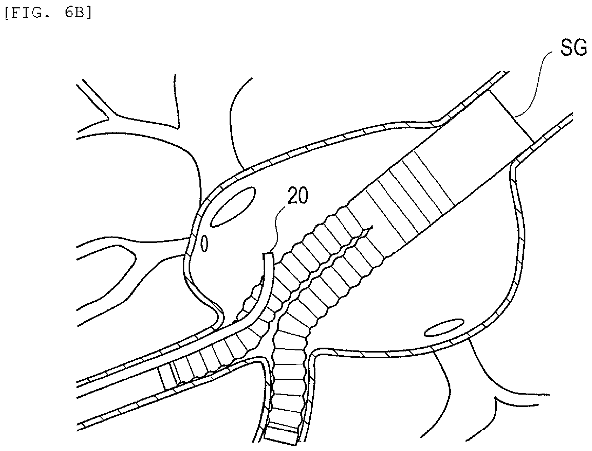

Next, as shown in FIG. 6B, the surgeon inserts, from the delivery catheter 20 into the living body lumen, a catheter (stent graft device) into which a stent graft SG is compressed and inserted, moves the catheter to an aneurysm target lesion using the guide wire GW, and deploys and indwells the stent graft SG from the catheter at the target lesion. In the deploying and indwelling step of the stent graft SG, first, a main body portion is deployed and indwelled, limb portions are attached to bifurcated portions of the main body portion, respectively, and the process ends.

### (Step 3: Loading Step)

Next, the surgeon loads the embolus 10 into the delivery catheter 20 using the embolus filled catheter set 60. As a method of loading the embolus 10 into the delivery catheter 20, the embolus filled catheter set 60 in which the filling catheter main body 61a is filled with the embolus 10 may be prepared, and in a state in which the filling catheter main body 61a is inserted into the delivery catheter 20, the extrusion pusher 66 may be inserted into the filling catheter main body 61a via the hub 62 to push out the embolus 10 and load the embolus 10 into the lumen 21a of the main body 21. When the embolus 10 is directly pushed into the aneurysm from the filling catheter main body 61a, the loading step is skipped. In this case, by inserting the filling catheter main body 61a loaded with the embolus 10 into the delivery catheter 20, the embolus 10 is loaded (accommodated) in the delivery catheter 20 as in the loading step.

### (Step 4: Conveyance Step)

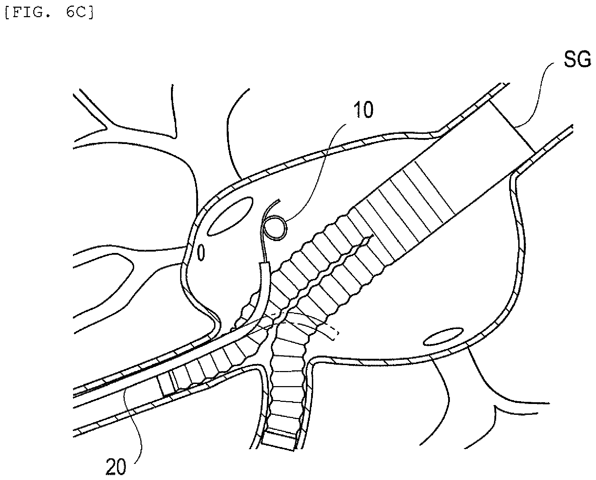

Next, as shown in FIG. 6C, the surgeon inserts the main body 41 of the delivery pusher 40 into the lumen 20a of the delivery catheter 20 loaded with the embolus 10 via the hub 22, and pushes the embolus 10 into the aneurysm. At this time, the surgeon conveys the embolus 10 to the indwelling position in the aneurysm by controlling the push-out direction of the embolus 10 while rotating the direction of the distal end of the curved portion 26 of the delivery catheter 20 in the rotation direction as shown in the drawing such that the embolus 10 does not enter a joint of the stent graft SG, a communication port of the side branch blood vessel, or the like. The surgeon repeats the loading step and the conveyance step such that an amount of the embolus 10 suitable for the patient is indwelled in the aneurysm while confirming a size of the aneurysm and an indwelling amount of the embolus 10. Further, as described in the loading step, when the embolus 10 is directly pushed out of the filling catheter main body 61a into the aneurysm to be indwelled, the embolus 10 can be pushed out by using the extrusion pusher 66 instead of the delivery pusher 40.

### (Step 5: Removal Step)

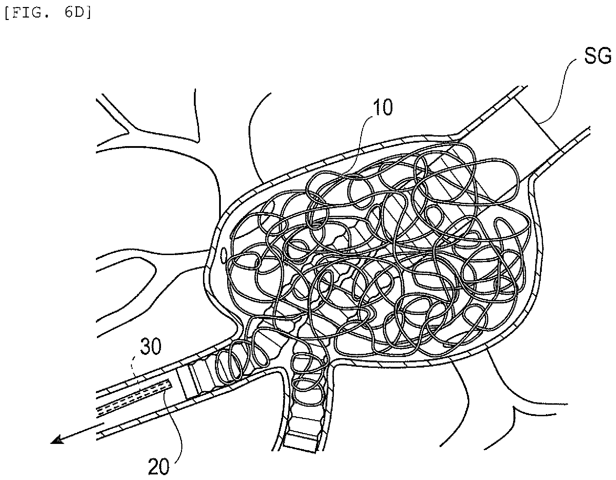

Thereafter, as shown in FIG. 6D, when the indwelling of the embolus 10 in the aneurysm is completed, the surgeon removes the delivery pusher 40 and the delivery catheter 20. At this time, since the curved state of the curved portion 26 can be corrected to the substantially straight state by inserting the correcting member 30 into the delivery catheter 20, a removal operation can be smoothly performed.

### (Step 6: Expansion and Closing Step)

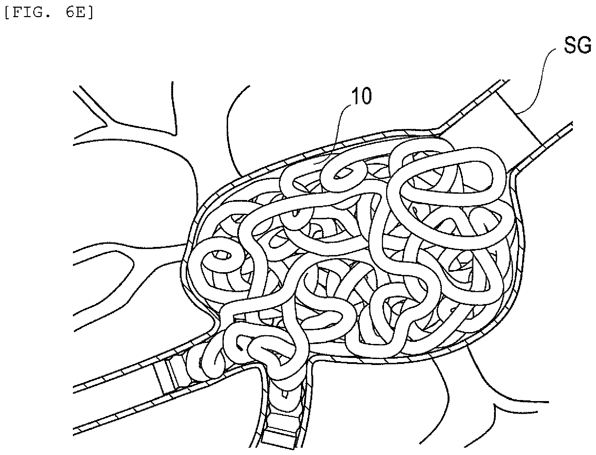

The embolus 10 indwelled in the aneurysm comes into contact with a liquid such as blood in the aneurysm and gradually swells, and as shown in FIG. 6E, the completely expanded embolus 10 fills a space between an inner surface of the aneurysm and an outer surface of the stent graft SG, thereby closing the aneurysm. Accordingly, the aneurysm is prevented from rupturing.

### [Functions and Effects]

As described above, the medical instrument set 100 according to the present embodiment includes: the elongated hollow delivery catheter 20 including the elongated main body 21, the curved portion 26 that is provided to extend to the distal end side of the main body 21 and curved so as to gradually move away from the central axis of the main body 21, and the lumen 20a that communicates from the distal end side of the curved portion 26 toward the proximal end side of the main body 21, and configured to deliver the embolus 10 into the aneurysm via the lumen 20a; and the elongated correcting member 30 configured to displace the curved portion 26 such that the amount of curvature of the curved portion 26 after the insertion becomes smaller than that before the insertion by being inserted into the lumen 20a of the delivery catheter 20.

The delivery system 200 according to the present embodiment includes the medical instrument set 100 according to the present embodiment, and the elongated delivery pusher 40 that pushes the embolus 10 loaded in the lumen 20a of the delivery catheter 20 out of the lumen 20a into the aneurysm.

Further, the embolus delivery medical system 300 according to the present embodiment includes the delivery system 200 according to the present embodiment, the embolus filled catheter 61 including the lumen for filling and insertable into the lumen 20a of the delivery catheter 20 in a state in which the lumen for filling is filled with the embolus 10, and the elongated extrusion pusher 66 that pushes the embolus 10 out of the lumen for filling.

With such a configuration, by inserting the correcting member 30 at the time of insertion or removal of the delivery catheter 20, the curved state of the curved portion 26 is corrected to the substantially straight state, and the insertion and removal operation of the delivery catheter 20 can be smoothly performed. Therefore, since the curved portion 26 is provided on the distal end side of the delivery catheter 20, the embolus 10 can be indwelled at the appropriate position by operating the delivery pusher 40 in a state in which the delivery catheter 20 is rotated by a predetermined amount in the rotation direction in accordance with the situation (the state of the aneurysm, the implantation position of the stent graft SG, the branched state of the side branch blood vessel, and the like) of the surgery. Therefore, it is possible to prevent the embolus 10 from entering slight gaps generated at connection portions between the bifurcated portions and the limb portions of the main body portion in the stent graft SG used in the stent graft interpolation, or the side branch blood vessel branched from the aneurysm. Further, since the embolus delivery medical system 300 includes the embolus filled catheter 61 and the extrusion pusher 66, even the relatively elongated embolus 10 can be smoothly loaded into the delivery catheter 20.

### [Other Embodiments]

The invention is not limited to the above-described embodiment, and for example, as to be described below, the invention can be appropriately modified and implemented in accordance with use environments or the like. In addition, the following modifications may be implemented in any combination without departing from the scope of the invention.

### <First Modification>

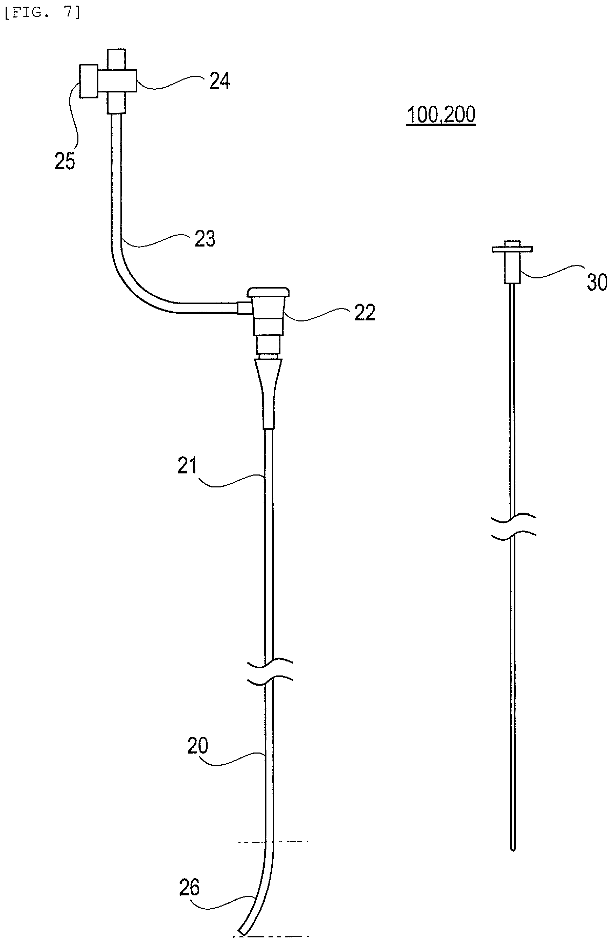

The correcting member 30 according to a first modification may be used as the delivery pusher 40. That is, as shown in FIG. 7, the medical instrument set 100 according to the present embodiment includes the delivery catheter 20 and the correcting member 30 that covers a function of the delivery pusher 40, and a configuration of the delivery pusher 40 in the delivery system 200 is deleted.

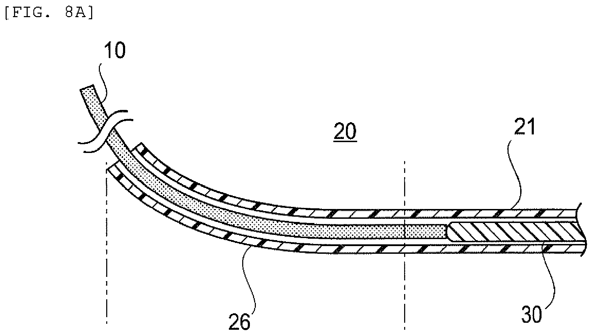

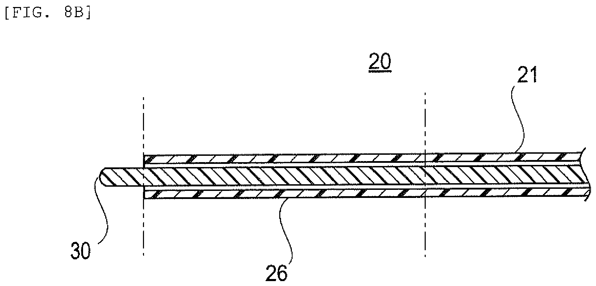

When the embolus 10 is conveyed by the correcting member 30 according to the first modification, as shown in FIG. 8A, the correcting member 30 functions as the delivery pusher 40 before reaching the curved portion 26 with the amount of curvature of the curved portion 26 being not corrected, and pushes the embolus 10 loaded in the delivery catheter 20 into the aneurysm. Then, when the correcting member 30 reaches the curved portion 26, as shown in FIG. 8B, the amount of curvature is displaced such that the curved state of the curved portion 26 becomes the substantially straight state.

In the first modification, when the correcting member 30 reaches the curved portion 26, the embolus 10 is already pushed into the aneurysm, leading to a final stage of the conveyance step, and therefore even if the curved portion 26 is corrected, the indwelling of the embolus 10 is hardly affected.

As described above, according to the correcting member 30 of the first modification, since the conveyance of the embolus 10 and the correction of the curved portion 26 to the substantially straight state can be performed by one member, the number of components is reduced and a system configuration becomes compact. In addition, since the curved state of the curved portion 26 is corrected to the substantially straight state at a timing at which the indwelling of the embolus 10 is finished, the delivery catheter 20 can be removed immediately. Therefore, after the conveyance of the embolus 10 by the delivery pusher 40 is finished, it is not necessary to replace the delivery pusher 40 with the correcting member 30 and remove the delivery catheter 20, and a removal operation of the delivery catheter 20 is simplified.

### <Second Modification>

The correcting member 30 according to a second modification may displace only an amount of curvature of the curved portion 26 when being inserted into the delivery catheter 20. That is, the correcting member 30 only needs to correct at least the amount of curvature of a region where the curved portion 26 exists in the delivery catheter 20.

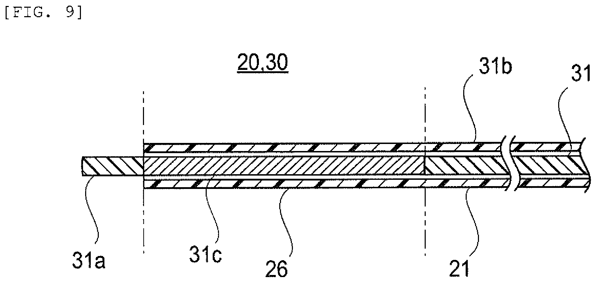

As a method for implementing such a function, as shown in FIG. 9, the correcting member 30 may be provided with a curvature correcting member 31c for correcting a curved state of the curved portion 26 to a substantially straight state between a distal end portion 31a and a proximal end portion 31b of a main body 31.

The curvature correcting member 31c in the correcting member 30 may be disposed in a region where the amount of curvature can be displaced such that at least a degree of curvature of the curved portion 26 becomes the substantially straight state when the correcting member 30 is inserted into the delivery catheter 20. Therefore, in FIG. 9, the curvature correcting member 31c is provided between the distal end portion 31a and the proximal end portion 31b, and the curvature correcting member 31c may be set as the distal end portion 31a. In this case, in FIG. 9, the distal end portion 31a is omitted, and the curvature correcting member 31c becomes the distal end portion 31a.

The curvature correcting member 31c is made of a material harder than at least a constituent material of the curved portion 26 such that the curved state of the curved portion 26 can be corrected. Therefore, in the correcting member 30, materials used for the distal end portion 31a and the proximal end portion 31b may be different from the material used for the curvature correcting member 31c. For example, the distal end portion 31a and the proximal end portion 31b can use the constituent material of the delivery pusher 40 described above, and the constituent material of the curvature correcting member 31c can use the same constituent material as the constituent material of the correcting member 30 described above.

Further, in order to improve ease of the insertion and removal operation of the delivery catheter 20, the distal end portion 31a and the proximal end portion 31b of the correcting member 30 may be made of a material softer than that of the curvature correcting member 31c. Accordingly, while the curved state of the curved portion 26 is displaced to the substantially straight state, another regions of the delivery catheter 20 can be flexibly deformed along a shape of a living body lumen.

As shown in FIG. 9, when the correcting member 30 according to the second modification is inserted into the delivery catheter 20 and the curvature correcting member 31c reaches an existence region of the curved portion 26, the amount of curvature of the curved portion 26 is corrected by the curvature correcting member 31c such that the curved state becomes the substantially straight state.

As described above, in the correcting member 30 according to the second modification, since the curvature correcting member 31c is set in the region where the curved portion 26 exists between the distal end portion 31a and the proximal end portion 31b of the main body 31, in the delivery catheter 20, while the curved state of the curved portion 26 is displaced to the substantially straight state, another regions of the delivery catheter 20 can be flexibly deformed along the shape of the living body lumen. Therefore, when a surgeon removes the delivery catheter 20 in a state in which the curved portion 26 is corrected by the curvature correcting member 31c, the surgeon can smoothly perform a removal operation without the curved portion 26 being caught in the living body lumen.

The medical instrument set 100, the delivery system 200, and the embolus delivery medical system 300 according to the present embodiment may be configured by combining the first and second modifications described above. In this case, the correcting member 30 functions as the delivery pusher 40, and the curvature correcting member 31c is provided between the distal end portion 31a and the proximal end portion 31b of the main body 31 of the correcting member 30. Accordingly, the correcting member 30 can be configured to achieve respective operational effects obtained in the first and second modifications described above.

This application is based on Japanese Patent Application 2020-062225, filed on March 31, 2020, the disclosure of which is incorporated as a whole by reference.

### Reference Signs List

10 embolus
20 delivery catheter (20a lumen)
21 main body (21a lumen) of delivery catheter
26 curved portion (26a lumen)
30 correcting member
31 main body (31a distal end portion, 31b proximal end portion, 31c curvature correcting member) of correcting member
40 delivery pusher
50 insertion assisting member
60 embolus filled catheter set
100 medical instrument set
200 delivery system
300 embolus delivery medical system

## Claims

1. A medical instrument set comprising:
an elongated hollow delivery catheter including an elongated main body, a curved portion that is provided to extend to a distal end side of the main body and curved so as to gradually move away from a central axis of the main body, and a lumen that communicates from a distal end of the curved portion toward a proximal end of the main body, and configured to deliver an embolus into an aneurysm via the lumen; and
an elongated correcting member configured to displace the curved portion such that an amount of curvature of the curved portion after the insertion becomes smaller than that before the insertion by being inserted into the lumen of the delivery catheter.

2. The medical instrument set according to claim 1, wherein
when the correcting member is inserted into the lumen of the delivery catheter, the correcting member displaces the amount of curvature of the curved portion such that the curved portion is in a substantially straight state.

3. The medical instrument set according to claim 1 or 2, wherein
the correcting member includes a curvature correcting member configured to displace only the amount of curvature of the curved portion when the correcting member is inserted into the lumen of the delivery catheter.

4. The medical instrument set according to any one of claims 1 to 3, further comprising:
an elongated insertion assisting member configured to assist delivery of the delivery catheter into the aneurysm by being assembled into the lumen of the delivery catheter, wherein
the insertion assisting member includes a guide wire lumen that communicates from a distal end to a proximal end and has a diameter smaller than that of the lumen of the delivery catheter.

5. The medical instrument set according to claim 4, wherein
the insertion assisting member functions as the correcting member.

6. A delivery system comprising:
the medical instrument set according to any one of claims 1 to 5; and
an elongated delivery pusher configured to push the embolus loaded in the lumen of the delivery catheter out of the lumen into the aneurysm.

7. A delivery system comprising:
the medical instrument set according to any one of claims 1 to 5, wherein
the correcting member functions as an elongated delivery pusher configured to push the embolus loaded in the lumen of the delivery catheter out of the lumen into the aneurysm.

8. An embolus delivery medical system comprising:
the delivery system according to claim 6 or 7;
an embolus filled catheter including a lumen for filling and insertable into the lumen of the delivery catheter in a state in which the lumen for filling is filled with the embolus; and
an elongated extrusion pusher configured to push the embolus out of the lumen for filling.

9. The embolus delivery medical system according to claim 8, wherein
the extrusion pusher functions as the delivery pusher.
